# DEMANDE DE BREVET EUROPEEN

(11) **EP 2 011 473 A1**
(43) Date de publication de la demande: **07.01.2009**
(21) Numéro de dépôt: 08159060.6
(22) Date de dépôt: 26.06.2008
(51) Int. Cl.: A61K 8/22, A61K 8/25, A61K 8/81, A61Q 5/08

(54) **Composition anhydre sous forme de pâte pour la décoloration des fibres kératiniques**

(30) Priorité: 29.06.2007 FR 0756184
(71) Demandeur: L'Oreal, 75008 Paris (FR)
(72) Inventeur: De La Mettrie, Roland, 78110, Le Vesinet (FR); Boche, Benoît, 92250, La Garenne Colombes (FR)
(74) Mandataire: Martin-Charbonneau, Virginie

(57) **Abrégé**

La présente invention a pour objet une composition anhydre sous forme de pâte pour la décoloration des fibres kératiniques, comprenant un ou plusieurs sels peroxygénés, un ou plusieurs agents alcalins, et un ou plusieurs complexes de peroxyde d'hydrogène et d'un polymère comprenant à titre de monomère au moins un monomère hétérocyclique vinylique.

Cette composition est utilisée pour la préparation d'une composition de décoloration prête à l'emploi, par mélange avec une composition aqueuse contenant ou non du peroxyde d'hydrogène.

## Description

La présente invention a pour objet une composition anhydre sous forme de pâte pour la décoloration des fibres kératiniques, et en particulier des fibres kératiniques humaines telles que les cheveux, comprenant un ou plusieurs sels peroxygénés, un ou plusieurs agents alcalins et un ou plusieurs complexes de peroxyde d'hydrogène et d'un polymère comprenant à titre de monomère au moins un monomère hétérocyclique vinylique.

L'invention concerne également le procédé de décoloration des fibres kératiniques humaines mettant en oeuvre une telle composition.

La décoloration des fibres kératiniques humaines et plus particulièrement des cheveux se fait par oxydation du pigment "mélanine" aboutissant à la solubilisation et l'élimination partielle ou totale de ce pigment.

Pour décolorer les cheveux, on utilise en général des poudres décolorantes contenant un réactif peroxygéné tel que les persulfates, perborates et percarbonates d'ammonium ou de métaux alcalins, que l'on associe au moment de l'emploi à une composition aqueuse de peroxyde d'hydrogène (ou « eau oxygénée »).

Les sels peroxygénés et le peroxyde d'hydrogène étant relativement stables en milieu acide, il est souvent nécessaire de les activer à pH basique pour obtenir une formation adéquate d'oxygène. I1 est donc usuel d'ajouter aux poudres décolorantes, des composés alcalins tels que l'urée, les silicates et les phosphates de métaux alcalins ou alcalino-terreux, et en particulier les métasilicates de métaux alcalins, ou des agents précurseurs d'ammoniac comme les sels d'ammonium.

Les poudres décolorantes ayant tendance à former de la poussière durant leur manutention, leur transport et leur stockage, et les produits qui les composent (persulfates, silicates alcalins) étant corrosifs, irritants pour les yeux, pour les voies respiratoires et les muqueuses, on a plus récemment développé des pâtes qui comprennent plus particulièrement lesdits agents pulvérulents dans un support liquide inerte organique épaissi. De telles compositions sont notamment décrites dans les demandes de brevets DE-3814 356 A1, DE-197 23 538 C₁ et le brevet américain N° 4 170 637.

Les poudres et les pâtes de décoloration sont classiquement mélangées, juste avant leur emploi, à une solution aqueuse de peroxyde d'hydrogène.

Ainsi, la demande de brevet US 2006/0254001 décrit une composition oxydante utilisable notamment en décoloration, qui est obtenue par mélange :
- d'une première composition (A) anhydre solide ou pâteuse, contenant un agglomérat d'agent alcalin et d'agent chélatant destiné à stabiliser le peroxyde d'hydrogène, et
- d'une seconde composition (B) aqueuse ou hydro-alcoolique, l'une et/ou l'autre des deux compositions (A) et (B) contenant du peroxyde d'hydrogène en solution ou sous forme d'un produit solide d'addition de peroxyde d'hydrogène avec un composé organique ou inorganique.

De préférence, seule la composition liquide (B) contient du peroxyde d'hydrogène.

Par ailleurs, la demande WO 93/14024 décrit des compositions de décoloration capillaire sous forme de poudre, contenant entre autre du persulfate d'ammonium ou de potassium et du carbonate de sodium, auxquels sont ajoutés au moment de l'emploi 5% en poids d'un complexe de polyvinylpyrrolidone et de peroxyde d'hydrogène, ainsi que 20 volumes d'une crème développante à base de peroxyde d'hydrogène aqueux.

Les compositions pulvérulentes à base de persels posent néanmoins des problèmes d'instabilité d'une part, et d'innocuité d'autre part, liée à la volatilité des poudres.

Par ailleurs, l'emploi des compositions aqueuses de peroxyde d'hydrogène peut également poser problème. En effet, ces compositions sont relativement agressives pour la peau, et surtout les yeux et les muqueuses, avec lesquels il convient d'éviter tout contact.

Ces problèmes se posent d'autant plus que la concentration en peroxyde d'hydrogène de la composition aqueuse est élevée.

I1 existe donc un besoin de diminuer les quantités ou les concentrations des solutions de peroxyde d'hydrogène employées, voire même de se passer totalement des solutions de peroxyde d'hydrogène

La Demanderesse a maintenant découvert que l'emploi, dans les pâtes de décoloration anhydres, d'un complexe tel que décrit ci-après permettait de résoudre ces problèmes.

La présente invention a donc pour objet une composition anhydre sous forme de pâte pour la décoloration des fibres kératiniques, comprenant un ou plusieurs sels peroxygénés, un ou plusieurs agents alcalins et un ou plusieurs complexes de peroxyde d'hydrogène et d'un polymère comprenant à titre de monomère au moins un monomère hétérocyclique vinylique.

La composition de décoloration selon l'invention présente l'avantage de pouvoir être employée en mélange avec une composition aqueuse comprenant ou non du peroxyde d'hydrogène.

Lorsqu'elle est employée sans peroxyde d'hydrogène, la composition selon l'invention est diluée au moment de son emploi avec une composition aqueuse ne contenant pas de peroxyde d'hydrogène, et notamment une composition aqueuse non oxydante. De manière particulièrement avantageuse, cette composition aqueuse peut être l'eau, ce qui permet de simplifier le mode opératoire et le conditionnement de la composition de décoloration, et de réduire les coûts.

Lorsqu'elle est employée avec une composition aqueuse de peroxyde d'hydrogène telle qu'une solution de peroxyde d'hydrogène dans l'eau, la composition selon l'invention présente l'avantage de permettre de diminuer la concentration de la solution de peroxyde d'hydrogène, tout en obtenant un éclaircissement de très bonne qualité.

Si une solution de concentration normale en peroxyde d'hydrogène est néanmoins employée, la composition selon l'invention présente l'avantage supplémentaire de permettre l'obtention d'un éclaircissement supérieur à celui qui serait obtenu avec une pâte classique ne contenant pas de complexe de polymère et de peroxyde d'hydrogène, et ce sans dégradation supplémentaire de la fibre kératinique. A l'inverse, si l'on augmente la quantité de persulfates dans une pâte de décoloration classique, ou que l'on augmente la concentration de la solution de peroxyde d'hydrogène, on risque de dégrader les fibres kératiniques lors du traitement de décoloration.

La présente invention a également pour objet l'utilisation de la composition anhydre sous forme de pâte selon l'invention, pour la préparation d'une composition de décoloration prête à l'emploi.

Par "composition prête à l'emploi" on entend, au sens de l'invention, la composition destinée à être appliquée telle quelle sur les fibres kératiniques, c'est à dire qu'elle résulte du mélange extemporané de la composition anhydre sous forme de pâte et de la composition aqueuse contenant ou non du peroxyde d'hydrogène.

L'invention vise également un procédé de décoloration des fibres kératiniques humaines, et plus particulièrement des cheveux, utilisant la composition de décoloration prête à l'emploi selon l'invention.

D'autres caractéristiques, aspects, objets et avantages de l'invention apparaîtront encore plus clairement à la lecture de la description et des exemples qui suivent.

Par anhydre, on entend, au sens de l'invention, une composition dont la teneur en eau est inférieure à 1%, et de préférence inférieure à 0,5% en poids, par rapport au poids total de la composition.

Par pâte, on entend au sens de l'invention une composition présentant une consistance intermédiaire entre un solide et un liquide et plus particulièrement une viscosité supérieure à 5 poises et de préférence à 10 poises à 25°C et à un taux de cisaillement de 1 s⁻¹. Cette viscosité peut être mesurée à l'aide d'un rhéomètre ou d'un viscosimètre cône-plan.

La composition selon l'invention contient un ou plusieurs sels peroxygénés, qui peuvent être avantageusement choisis parmi les persulfates, les perborates et les percarbonates d'ammonium ou de métaux alcalins ainsi que le peroxyde de magnésium et les mélanges de ces composés.

On utilise de préférence les persulfates, et parmi ceux-ci principalement les persulfates de sodium et de potassium.

Dans la composition selon l'invention, le ou les sel(s) peroxygéné(s) peuvent être présents à une concentration allant de 10 à 70% en poids, et de préférence de 20 à 60% en poids, par rapport au poids total de la composition.

La composition selon l'invention contient un ou plusieurs agents alcalins, qui peuvent être avantageusement choisis parmi l'ammoniaque, les silicates, les métasilicates, les phosphates, les hydrogénophosphates,les carbonates et les hydrogénocarbonates de métaux alcalins ou alcalino-terreux, et en particulier les métasilicates de métaux alcalins.

Le ou les agent(s) alcalin(s) peuvent être présents dans la composition selon l'invention à une concentration allant de 0,01 à 40% en poids, et de préférence de 0,1 à 30% en poids, par rapport au poids total de la composition.

Selon une alternative, ils peuvent être présents dans une composition aqueuse à mélanger au moment de l'emploi à la composition selon l'invention.

Selon un mode de réalisation préféré, la composition selon l'invention contient également un ou plusieurs sels d'ammonium, tels que par exemple le chlorure d'ammonium, le sulfate d'ammonium, le phosphate d'ammonium ou le nitrate d'ammonium. Le ou les sels d'ammonium sont alors avantageusement présents dans la composition selon l'invention à une concentration allant de 0,01 à 40% en poids, de préférence de 0,1 à 30% en poids, par rapport au poids total de la composition.

La composition selon l'invention comprend en outre un ou plusieurs complexes de peroxyde d'hydrogène et d'un polymère contenant à titre de monomère au moins un monomère hétérocyclique vinylique.

Plus particulièrement, le monomère hétérocyclique vinylique est choisi parmi les monomères comprenant un hétérocycle ayant 4 à 6 chaînons, éventuellement condensé à un cycle benzénique et comporte de 1 à 4 hétéroatomes intracycliques identiques ou non ; le nombre d'hétéroatomes intracycliques étant inférieur à celui des chaînons de l'hétérocycle. De préférence, le nombre d'hétéroatomes intracycliques est de 1 ou 2.

Plus particulièrement encore, le ou les hétéroatomes sont choisis parmi le soufre, l'oxygène, l'azote, de préférence parmi l'azote et l'oxygène. Conformément à un mode de réalisation encore plus avantageux de l'invention, le monomère comprend au moins un atome d'azote intracyclique.

L'hétérocycle vinylique peut éventuellement être substitué par un ou plusieurs groupements alkyle en C₁-C₄, de préférence en C₁-C₂.

De préférence, le monomère hétérocyclique est choisi parmi les monomères N-vinyliques.

Parmi les monomères envisageables, on peut citer les monomères suivants, éventuellement substitués : la N-vinylpyrrolidone, le vinylcaprolactame, la N-vinylpipéridone, la N vinyl 3-morpholine, la N-vinyl-4-oxazolinone, la 2-vinylpyridine, la 4-vinylpyridine, la 2-vinylquinoline, le 1-vinylimidazole, le 1-vinylcarbazole. De préférence, le monomère est la N-vinylpyrrolidone éventuellement substituée.

Conformément à un mode de réalisation particulièrement avantageux de l'invention, le polymère est un homopolymère.

Il n'est cependant pas exclu de mettre en oeuvre un copolymère. Dans un tel cas, le ou les comonomères sont choisis parmi l'acétate de vinyle, les acides (méth)acryliques, les amides (méth)acryliques, les esters d'alkyle en C₁-C₄ d'acide (méth)acrylique substitués ou non.

Le polymère intervenant dans ce complexe peut de plus être soluble ou insoluble dans l'eau. De préférence, il est soluble dans l'eau. Il peut présenter des poids moléculaires moyens variables, de préférence compris entre 10³ et 3.10⁶ g/mol, de préférence entre 10³ et 2.10⁶ g/mol. Il est également possible d'employer des mélanges de tels polymères.

Des complexes de H₂O₂ avec ce type de polymères sont notamment décrits dans les brevets EP832846, EP 714919, DE4344131, DE19545380.

De manière avantageuse, ledit complexe comprend de 10 à 30 % en poids de peroxyde d'hydrogène, plus particulièrement de 13 à 25 % en poids et de préférence de 18 à 22% en poids, par rapport au poids total du complexe.

Selon une variante encore plus avantageuse de l'invention, dans ce complexe, le rapport molaire entre le ou les monomères hétérocycliques vinyliques et le peroxyde d'hydrogène va de 0,5 à 2, de préférence de 0,5 à 1.

Ce complexe se présente avantageusement sous forme d'une poudre substantiellement anhydre, c'est-à-dire contenant moins de 5% en poids d'eau. Il peut être préparé de manière connue en soi, par exemple telle que décrite dans le brevet US 5 008 106 ou encore US 5 077 047.

A titre d'exemples de composés de ce type, on peut citer par exemple les produits du type Peroxydone K-30, Peroxydone K-90, Peroxydone XL-10 ainsi que les complexes formés avec le peroxyde d'hydrogène et l'un des polymères suivants de type Plasdone K-17, Plasdone K-25, Plasdone K-29/32, Plasdone K-90, Polyplasdone INF-10, Polyplasdone XL-10, Polyplasdone XL, Plasdone S-630, Styleze 2000 Terpolymer, série des Ganex copolymers, commercialisés par la société ISP.

La composition anhydre selon l'invention contient avantageusement de 0,1 à 50 % en poids de complexe de polymère contenant à titre de monomère au moins un monomère hétérocyclique vinylique et de peroxyde d'hydrogène, de préférence de 0,1 à 30 % en poids, de manière encore plus préférée de 1 à 25 % en poids, par rapport au poids total de la composition.

La composition selon l'invention contient par ailleurs de préférence un ou plusieurs liquides inertes organiques, qui peuvent être avantageusement choisis dans le groupe formé par les polydécènes de formule C₁₀ₙH_{[(20n)+2]} avec n variant de 3 à 9, les esters d'alcools gras ou d'acides gras, les esters ou di-esters de sucres et d'acides gras en C₁₂-C₂₄, les éthers cycliques ou les esters cycliques, les huiles de silicone, les huiles minérales ou les huiles végétales, et leurs mélanges.

Par liquide inerte au sens de la présente invention, on entend un composé capable d'écoulement à température ambiante, généralement entre 15°C et 40°C, et à pression atmosphérique, sous l'action de son propre poids.

Le terme inerte signifie que le liquide ne réagit pas au moins dans les conditions de stockage, avec les ingrédients de la composition.

A titre d'exemples de liquide inerte, on peut citer les polydécènes de formule C₁₀ₙH_{[(20n)+2]} dans laquelle n varie de 3 à 9 et de préférence de 3 à 7, les esters et en particulier les esters d'alcools gras ou d'acides gras, les esters ou di-esters de sucres d'acides gras en C₁₂-C₂₄, ou les esters cycliques, les éthers cycliques, les huiles de silicone, les huiles minérales, les huiles végétales ou les huiles animales, ou leurs mélanges.

Les composés de formule C₁₀ₙH_{[(20n)+2]} avec n variant de 3 à 9 répondent à l'appellation "polydécène" du Dictionnaire CTFA 7ème édition 1997 de la Cosmetic, Toiletry and Fragrance Association, USA, ainsi qu'à la même appellation I.N.C.I. aux USA et en Europe. Ce sont des produits d'hydrogénation des poly-1-décènes.

Parmi ces composés, on préfère selon l'invention ceux pour lesquels dans la formule, n varie de 3 à 7.

On peut citer à titre d'exemple le produit vendu sous la dénomination Silkflo^{®} 366 NF Polydecene par la société Amoco Chemical, ceux vendus sous la dénomination Nexbase® 2002 FG, 2004 FG, 2006 FG et 2008 FG par la société Fortum.

En ce qui concerne les esters on peut citer à titre d'exemple :
- les esters de monoalcools inférieurs saturés linéaires ou ramifiés en C₃-C₆, avec des acides gras monofonctionnels en C₁₂-C₂₄, ces derniers pouvant être linéaires ou ramifiés, saturés ou insaturés et choisis notamment parmi les oléates, laurates, palmitates, myristates, béhénates, cocoates, stéarates, linoléates, linolénates, caprates, arachidonates, ou leurs mélanges comme notamment les oléo-palmitates, oléo-stéarates, palmito-stéarates. Parmi ces esters, on préfère plus particulièrement utiliser le palmitate d'isopropyle, le myristate d'isopropyle, le stéarate d'octyl dodécyle et l'isononaoate d'isononyle.
- les esters de monoalcools linéaires ou ramifiés en C₃-C₈, avec des acides gras bifonctionnels en C₈-C₂₄, ces derniers pouvant être linéaires ou ramifiés, saturés ou insaturés, comme par exemple le di-ester isopropylique de l'acide sébacique, appelé aussi sébaçate de di-isopropyle,
- les esters de monoalcools linéaires ou ramifiés en C₃-C₈, avec des acides bifonctionnels en C₂-C₈, ces derniers pouvant être linéaires ou ramifiés, saturés ou insaturés, comme par exemple l'adipate de di-octyle et le maléate de di-caprylyle,
- l'ester d'un acide trifonctionnnel comme le citrate de tri-éthyle.

En ce qui concerne les esters et di-esters de sucres d'acides gras en C₁₂-C₂₄, on entend par "sucre" des composés qui possèdent plusieurs fonctions alcool, avec ou sans fonction aldéhyde ou cétone, et qui comportent au moins 4 atomes de carbone. Ces sucres peuvent être des monosaccharides, des oligosaccharides ou des polysaccharides.

Comme sucres utilisables selon l'invention, on peut citer par exemple le sucrose (ou saccharose), le glucose, le galactose, le ribose, le fucose, le maltose, le fructose, le mannose, l'arabinose, le xylose, le lactose, et leurs dérivés notamment alkylés, tels que les dérivés méthylés comme le méthylglucose.

Les esters de sucres et d'acides gras utilisables selon l'invention peuvent être choisis notamment dans le groupe comprenant les esters ou mélanges d'esters de sucres décrits ci-avant et d'acides gras en C₁₂-C₂₄, linéaires ou ramifiés, saturés ou insaturés.

Les esters peuvent être choisis parmi les mono-, di-, tri- et tétra-esters, les polyesters et leurs mélanges.

Ces esters peuvent être par exemple choisis parmi les oléates, laurates, palmitates, myristates, béhénates, cocoates, stéarates, linoléates, linolénates, caprates, arachidonates, ou leurs mélanges comme notamment les esters mixtes oléo-palmitates, oléo-stéarates, palmito-stéarates.

Plus particulièrement, on préfère utiliser les mono- et di- esters et notamment les mono- ou di- oléates, stéarates, béhénates, oléopalmitates, linoléates, linolénates, oléostéarates, de saccharose, de glucose ou de méthylglucose.

On peut citer à titre d'exemple le produit vendu sous la dénomination Glucate® DO par la société Amerchol, qui est un dioléate de méthylglucose.

On peut aussi citer à titre d'exemples d'esters ou de mélanges d'esters de sucre d'acide gras :
- les produits vendus sous les dénominations F160, F140, F110, F90, F70, SL40 par la société Crodesta, désignant respectivement les palmito-stéarates de sucrose formés de 73 % de monoester et 27 % de di-et tri-ester, de 61 % de monoester et 39 % de di-, tri-, et tétra-ester, de 52 % de monoester et 48 % de di-, tri-, et tétra-ester, de 45 % de monoester et 55 % de di-, tri-, et tétra-ester, de 39 % de monoester et 61 % de di-, tri-, et tétra-ester, et le mono-laurate de sucrose;
- les produits vendus sous la dénomination Ryoto Sugar Esters par exemple référencés B370 et correspondant au béhénate de saccharose formé de 20 % de monoester et 80 % de di-triester-polyester;
- le mono-di-palmito-stéarate de sucrose commercialisé par la société Goldschmidt sous la dénomination Tegosoft® PSE.

En ce qui concerne les éthers cycliques et esters cycliques, conviennent notamment la γ-butyrolactone, le diméthyl isosorbide, ou le diisopropyl isosorbide.

Les huiles de silicone peuvent aussi être employées comme liquide organique inerte.

Plus particulièrement, les huiles de silicone convenables sont des fluides de silicones liquides de viscosité inférieure ou égale à 10 000 mPa.s à 25 °C, la viscosité des silicones étant mesurée selon la norme ASTM 445 Appendice C.

Les huiles de silicone sont définies plus en détail dans l'ouvrage de Walter NOLL "Chemistry and Technology of Silicones" (1968) - Academic Press.

Parmi les huiles de silicone utilisables selon l'invention, on peut citer notamment les huiles de silicones vendues sous les dénominations DC-200 fluid - 5 mPa.s, DC-200 fluid - 20 mPa.s, DC-200 fluid - 350 mPa.s, DC-200 fluid - 1000 mPa.s, DC-200 fluid - 10 000 mPa.s, DC-8566 amino fluid, DC 245 fluid, par la société Dow Corning.

Les huiles minérales peuvent aussi être utilisées en tant que liquide inerte organique, comme par exemple l'huile de paraffine, la vaseline.

Les huiles végétales peuvent aussi convenir, et notamment l'huile d'avocat, l'huile d'olive ou la cire liquide de jojoba, l'huile de camellia, de même que les huiles animales telles que la lanoline.

On peut encore utiliser des solvants apolaires comme notamment les dérivés dicapryliques apolaires, et plus particulièrement le dicapryl carbonate, le dicaprylether.

De préférence, le liquide inerte organique est choisi parmi les polydécènes de formule C₁₀ₙH_{[(20n)+2]} dans laquelle n varie de 3 à 9 et de préférence de 3 à 7, les esters d'alcools gras ou d'acides gras, l'huile de vaseline, l'huile de paraffine, et leurs mélanges.

Dans les compositions selon l'invention, le ou les liquide(s) inerte(s) organique(s) sont de préférence présents à une concentration allant de 15 à 35% et de préférence allant de 18 à 30% en poids par rapport au poids total de la composition anhydre.

La composition selon l'invention peut également comprendre au moins un polymère amphiphile non ionique comportant au moins une chaîne grasse.

De tels polymères sont choisis de préférence parmi :
- (1) les celluloses modifiées par des groupements comportant au moins une chaîne grasse ; on peut citer à titre d'exemple :
   - les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en C₈-C₂₂, comme le produit NATROSOL PLUS GRADE 330 CS (alkyles en C₁₆) vendu par la société AQUALON, ou le produit BERMOCOLL EHM 100 vendu par la société BEROL NOBEL,
   - celles modifiées par des groupes polyalkylène glycol éther d'alkyl phénol, tel que le produit AMERCELL POLYMER HM-1500 (polyéthylène glycol (15) éther de nonyl phénol) vendu par la société AMERCHOL.
- (2) les hydroxypropylguars modifiés par des groupements comportant au moins une chaîne grasse en C₈ à C₂₂, tel que le produit JAGUAR XC-95/3 (chaîne alkyle en C₁₄) vendu par la société RHODIA, le produit ESAFLOR HM 22 (chaîne alkyle en C₂₂) vendu par la société LAMBERTI, les produits RE210-18 (chaîne alkyle en C₁₄) et RE205-1 (chaîne alkyle en C₂₀) vendus par la société RHONE POULENC.
- (3) les copolymères de vinyl pyrrolidone et de monomères hydrophobes à chaîne grasse dont on peut citer à titre d'exemple :
   - les produits ANTARON V216 ou GANEX V216 (copolymère vinylpyrrolidone / hexadécène) vendu par la société I.S.P.
   - les produits ANTARON V220 ou GANEX V220 (copolymère vinylpyrrolidone / eicosène) vendu par la société I.S.P.
- (4) les copolymères de méthacrylates ou d'acrylates d'alkyles en C₁-C₆ et de monomères amphiphiles comportant au moins une chaîne grasse.
- (5) les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.
- (6) les polymères à squelette aminoplaste éther possédant au moins une chaîne grasse, tels que les composés PURE THIX proposés par la société SUD-CHEMIE.
- (7) les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

De préférence, les polyéthers polyuréthanes comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.

Les polyéthers polyuréthanes non-ioniques à chaîne grasse peuvent être des copolymères triblocs dont la séquence hydrophile est une chaîne polyoxyéthylénée comportant de 50 à 1000 groupements oxyéthylénés. Les polyéthers polyuréthanes non-ioniques comportent une liaison uréthanne entre les séquences hydrophiles, d'où l'origine du nom.

Par extension figurent aussi parmi les polyéthers polyuréthanes non-ioniques à chaîne grasse, ceux dont les séquences hydrophiles sont liées aux séquences lipophiles par d'autres liaisons chimiques.

A titre d'exemples de polyéthers polyuréthanes non-ioniques à chaîne grasse utilisables dans l'invention, on peut citer le Ser-ad FX 1100 de la société SERVO DELDEN qui est un copolymère dénommé sous la désignation I.N.C.I. européenne et américaine "Steareth-100/PEG-136/H.M.D.I. Copolymer".

On peut aussi utiliser le Rhéolate 205 à fonction urée vendu par la société RHEOX ou encore les Rhéolates 208, 204 ou 212, ainsi que l'Acrysol RM 184.

On peut également citer le produit ELFACOS T210 à chaîne alkyle en C₁₂₋₁₄ et le produit ELFACOS T212 à chaîne alkyle en C₁₈ de chez AKZO.

Les polyéthers polyuréthanes utilisables selon l'invention sont en particulier ceux décrits dans l'article de G. Fonnum, J. Bakke et Fk. Hansen - Colloid Polym. Sci 271, 380.389 (1993).

On préfère en particulier les polyéthers polyuréthanes comportant au moins une chaîne grasse en C₁₀ à C₂₀, et les hydroxypropylguars modifiés par des groupements comportant au moins une chaîne grasse en C₈ à C₂₂.

La composition selon l'invention peut également comprendre au moins un polymère amphiphile anionique comportant au moins une chaîne grasse.

Les polymères amphiphiles anioniques comportant au moins une chaîne grasse utilisables selon la présente invention sont des polymères réticulés ou non réticulés, comprenant :
- des motifs hydrophiles dérivés d'un ou de plusieurs monomères à insaturation éthylénique portant une fonction acide carboxylique libre, ou une fonction sulfonique libre ou partiellement ou totalement neutralisée, et
- des motifs hydrophobes dérivés d'un ou de plusieurs monomères à insaturation éthylénique portant une chaîne latérale hydrophobe, et
   éventuellement
- des motifs de réticulation dérivés d'un ou plusieurs monomères polyinsaturés.

Le ou les monomères à insaturation éthylénique portant une fonction acide carboxylique sont choisis parmi l'acide éthacrylique, l'acide méthacrylique et l'acide acrylique, de préférence parmi l'acide méthacrylique, l'acide acrylique et des mélanges de ceux-ci.

Le ou les monomères à insaturation éthylénique portant une chaîne latérale hydrophobe peuvent être (i) des esters d'acides carboxyliques insaturés et d'alcools gras, ou (ii) des éthers d'allyle et d'alcools gras.
(i) Les esters d'acides carboxyliques insaturés et d'alcools gras sont choisis par exemple parmi les éthacrylates, méthacrylates et/ou acrylates d'alkyles en C₁₀₋₃₀, de préférence en C₁₂₋₂₂. Ils englobent par exemple l'acrylate de lauryle, l'acrylate de stéaryle, l'acrylate de décyle, l'acrylate d'isodécyle, l'acrylate de dodécyle, et les méthacrylates correspondants, à savoir le méthacrylate de lauryle, le méthacrylate de stéaryle, le méthacrylate de décyle, le méthacrylate d'isodécyle, et le méthacrylate de dodécyle.
(ii) Les éthers allyliques d'alcools gras formant les motifs hydrophobes des polymères amphiphiles anioniques de la présente invention correspondent à la formule (I) suivante :

   CH₂ = C R' CH₂ O Bₙ R (I)

   dans laquelle R' désigne H ou CH₃, B désigne le radical éthylèneoxy, n est nul ou désigne un entier allant de 1 à 100, R désigne un radical hydrocarboné choisi parmi les radicaux alkyle, arylalkyle, aryle, alkylaryle, cycloalkyle, comportant de 8 à 30 atomes de carbone, de préférence 10 à 24, et plus particulièrement encore de 12 à 18 atomes de carbone. Un motif de formule (I) plus particulièrement préféré est un motif dans lequel R' désigne H, n est égal à 10, et R désigne un radical stéaryle(C₁₈).

Ledit monomère réticulant est un composé comportant au moins deux doubles liaisons polymérisables non conjuguées l'une avec l'autre. On peut citer à tire d'exemple le phtalate de diallyle, le (méth)acrylate d'allyle, le divinylbenzène, le diméthacrylate de (poly)éthylèneglycol, le méthylène-bis-acrylamide, le polyallylsucrose ou le polyallylpentaérythritol.

Des polymères amphiphiles anioniques du type décrit ci-dessus sont décrits et préparés, par exemple dans les brevets US-3 915 921 et US-4 509 949 (copolymères d'acide (méth)acrylique et de (méth)acrylates d'alkyles en C₁₀₋₃₀, ou dans le brevet EP-0216479 B2 (copolymères d'acide (méth)acrylique et d'éthers allyliques d'alcools gras).

On peut citer à titre d'exemples de polymères préférés :
- les polymères réticulés d'acide acrylique et de méthacrylate d'alkyle en C₁₀₋₃₀, tels que le CARBOPOL ETD-2020 commercialisé par la société GOODRICH;
- les polymères réticulés d'acide acrylique et d'acrylate d'alkyle en C₁₀₋₃₀, tels que les polymères commercialisés sous les dénominations CARBOPOL 1382, PEMULEN TR1, PEMULEN TR2 par la société GOODRICH;
- le terpolymère acide méthacrylique/acrylate d'éthyle/méthacrylate de stéaryle oxyéthyléné (55/35/10);
- le terpolymère acide (méth)acrylique/acrylate d'éthyle/méthacrylate de béhényle oxyéthyléné 25 OE, et
- le terpolymère réticulé acide méthacrylique/acrylate d'éthyle/stéareth-10 allyl éther.

Les polymères amphiphiles anioniques comportant comme motifs hydrophiles au moins un monomère à insaturation éthylénique à groupement sulfonique, sous forme libre ou partiellement ou totalement neutralisée et au moins une partie hydrophobe sont par exemple décrits dans les demandes de brevets français de la demanderesse N°0016954 et 0100328 dont le contenu fait partie intégrante de la présente invention.

On peut notamment citer plus particulièrement parmi eux :
le copolymère acide acrylamido-2-méthyl-2-propane-sulfonique(AMPS)/n-dodécylacrylamide neutralisé par la soude, le copolymère réticulé par du méthylène-bis-acrylamide constitué de 75% en poids de motifs AMPS neutralisés par NH3 et de 25% en poids de motifs acrylate de GENAPOL T-250, le copolymère réticulé par du méthacrylate d'allyle constitué de 90% en poids de motifs AMPS neutralisés par NH3 et de 10% en poids de motifs méthacrylate de GENAPOL T-250, ou le copolymère réticulé par du méthacrylate d'allyle constitué de 80% en poids de motifs AMPS neutralisés par NH₃ et de 20% en poids de motifs méthacrylate de GENAPOL T-250.

Dans les compositions selon l'invention, le ou les polymères amphiphiles non ioniques et/ou anioniques comportant au moins une chaîne grasse peuvent être présents à une concentration allant de 0,01 à 30% et de préférence de 0,01 à 15% par rapport au poids total de la composition.

Les compositions selon l'invention peuvent comprendre en outre au moins un polymère épaississant hydrosoluble sans chaînes grasses.

Les polymères épaississants hydrosolubles utilisables dans les compositions selon l'invention englobent tous les polymères hydrosolubles synthétiques ou d'origine naturelle utilisés classiquement dans le domaine cosmétique et différents des polymères amphiphiles non ioniques et/ou anioniques comportant au moins une chaîne grasse décrits ci-dessus.

Comme exemples de polymères synthétiques, on peut citer, la polyvinylpyrrolidone, l'acide polyacrylique, le polyacrylamide, l'acide poly-2-acrylamidopropanesulfonique non réticulé comme par exemple le produit vendu sous la dénomination SIMUGEL EG par la société SEPPIC, l'acide poly-2-acrylamido-2-méthylpropane sulfonique réticulé, l'acide poly-2-acrylamido-2-méthylpropane sulfonique réticulé et partiellement neutralisé par l'ammoniaque vendu sous la marque HOSTACERIN AMPS par la société CLARIANT, des mélanges à effet épaississant synergique de l'acide poly-2-acrylamido-2-méthylpropane sulfonique non réticulé avec des éthers d'hydroxyalkylcellulose ou avec des poly(oxyde d'éthylène) tel qu'ils sont décrits dans le brevet US-4540510, ou des mélanges à effet épaississant synergique d'un acide poly(méth)acrylamido-alkyl(C1-C4)-sulfonique de préférence réticulé avec un copolymère réticulé de l'anhydride maléique et d'un alkyl(C₁-C₅)vinyléther tels que le mélange HOSTACERIN AMPS / STABILEZE QM (de la société ISF) et tels qu'ils sont décrits dans la demande de brevet français de la demanderesse N°0014416.

Les polymères épaississants d'origine naturelle utilisables selon la présente invention, sont de préférence des polymères comportant au moins un motif sucre, à savoir : les gommes de guar non-ioniques; les gommes de biopolysaccharides d'origine microbienne telles que les gommes de scléroglucane ou de xanthane; les gommes issues d'extrudats végétaux telles que les gommes arabique, ghatti, karaya, tragacanthe, carrageenane, agar et caroube; les pectines; les alginates; les amidons; les hydroxyalkyl(C₁-C₆)celluloses et carboxyalkyl(C₁-C₆)celluloses.

Par "motif sucre", on désigne ici une portion monosaccharidique (i.e. monosaccharide ou oside ou sucre simple) ou une portion oligosaccharidique (chaînes courtes formées de l'enchaînement d'unités monosaccharidiques, éventuellement différentes) ou une portion polysaccharidique [longues chaînes constituées d'unités monosaccharidiques, éventuellement différentes, i.e. polyholosides ou polyosides (homopolyosides ou hétéropolyosides)]. Les unités saccharidiques peuvent être en outre substituées par des radicaux alkyle, ou hydroxyalkyle, ou alcoxy, ou acyloxy, ou carboxyle, les radicaux alkyle comportant de 1 à 4 atomes de carbone.

Les gommes de guar non ioniques peuvent être modifiées ou non modifiées.

Les gommes de guar non modifiées sont par exemple les produits vendus sous la dénomination GUARGEL D/15 par la société GOODRICH, VIDOGUM GH 175 par la société UNIPECTINE, et sous les dénominations MEYPRO-GUAR 50 et JAGUAR C par la société MEYHALL.

Les gommes de guar non-ioniques modifiées peuvent être notamment modifiées par des groupements hydroxyalkyle en C₁-C₆.

Parmi les groupements hydroxyalkyle, on peut mentionner à titre d'exemple, les groupements hydroxyméthyle, hydroxyéthyle, hydroxypropyle et hydroxybutyle.

Ces gommes de guar sont bien connues de l'état de la technique et peuvent, par exemple être préparées en faisant réagir des oxydes d'alcènes correspondants, tels que par exemple des oxydes de propylène, avec la gomme de guar de façon à obtenir une gomme de guar modifiée par des groupements hydroxypropyle.

Le taux d'hydroxyalkylation, qui correspond au nombre de molécules d'oxyde d'alkylène consommées par le nombre de fonctions hydroxyle libres présentes sur la gomme de guar, varie de préférence de 0,4 à 1,2.

De telles gommes de guar non-ioniques éventuellement modifiées par des groupements hydroxyalkyle sont par exemple vendues sous les dénominations commerciales JAGUAR HP8, JAGUAR HP60 et JAGUAR HP120, JAGUAR DC 293 et JAGUAR HP 105 par la société RHONE POULENC (MEYHALL) ou sous la dénomination GALACTASOL 4H4FD2 par la société AQUALON.

Les gommes de biopolysaccharides d'origine microbienne telles que les gommes de scléroglucane ou de xanthane, les gommes issues d'extrudats végétaux telles que les gommes arabique, gomme ghatti, gomme karaya, tragacanthe, carrageenane, agar et caroube, les hydroxyalkylcelluloses et les carboxyméthylcelluloses, les pectines, les alginates et les amidons sont bien connus de l'homme de l'art et décrits notamment dans l'ouvrage de Robert L. DAVIDSON intitulé "Handbook of Water soluble gums and resins" édité chez Mc Graw Hill Book Company (1980).

Parmi ces gommes, les scléroglucanes sont représentés entre autres par les produits vendus sous la dénomination ACTIGUM CS par la société SANOFI BIO INDUSTRIES et en particulier ACTIGUM CS 11 et sous la dénomination AMIGEL par la société ALBAN MULLER INTERNATIONAL. D'autres scléroglucanes tels que celui traité au glyoxal dans la demande de brevet français N°2633940 peuvent également être utilisés.

Les xanthanes sont représentés entre autres par les produits vendus sous les dénominations KELTROL, KELTROL T, KELTROL TF, KELTROL BT, KELTROL RD, KELTROL CG par la société NUTRASWEET KELCO, ou sous les dénominations RHODICARE S, RHODICARE H par la société RHODIA CHIMIE.

Parmi les dérivés d'amidon, on peut citer par exemple le produit vendu sous la dénomination PRIMOGEL par la société AVEBE.

Les hydroxyalkyl(C1-C6)celluloses sont plus particulièrement des hydroxyéthylcelluloses telles que celles vendues sous les dénominations CELLOSIZE QP3L, CELLOSIZE QP4400H, CELLOSIZE QP30000H, CELLOSIZE HEC30000A, CELLOSIZE POLYMER PCG10, par la société AMERCHOL, ou NATROSOL 250HHR, NATROSOL 250MR, NATROSOL 250M, NATROSOL 250HHXR, NATROSOL 250HHX, NATROSOL 250HR, NATROSOL HX, par la société HERCULES, ou encore TYLOSE H1000 par la société HOECHST.

Les hydroxyalkyl(C₁-C₆)celluloses sont également plus particulièrement des hydroxypropylcelluloses comme les produits vendus sous les dénominations KLUCEL EF, KLUCEL H, KLUCEL LHF, KLUCEL MF, KLUCEL G, par la société AQUALON.

Parmi les carboxyalkyl(C₁-C₆)celluloses, on utilise de préférence la carboxyméthylcellulose dont on peut citer les produits vendus sous les dénominations BLANOSE 7M8/SF, BLANOSE RAFFINEE 7M, BLANOSE 7LF, BLANOSE 7MF, BLANOSE 9M31F, BLANOSE 12M31XP, BLANOSE 12M31P, BLANOSE 9M31XF , BLANOSE 7H, BLANOSE 7M31, BLANOSE 7H3SXF, par la société AQUALON, ou encore AQUASORB A500 et AMBERGUM 1221, par le société HERCULES, ou encore CELLOGEN HP810A et CELLOGEN HP6HS9, par la société MONTELLO, ou encore PRIMELLOSE par la société AVEBE.

Lorsqu'ils ils sont présents dans les compositions selon la présente invention, les polymères épaississants hydrosolubles sans chaînes grasses sont présents dans une proportion pondérale allant de 0,01 à 30% et de préférence de 0,01 à 15% par rapport au poids total de la composition.

La composition selon l'invention peut en outre contenir des cires hydrocarbonées, fluorées ou siliconées ou leurs mélanges. Les cires siliconées peuvent être des cires comportant une structure siliconée et des motifs à une ou plusieurs chaînes alkyles ou alcoxy pendantes et/ou en bout de structure siliconée, ces chaînes étant linéaires ou ramifiées et comportant de 10 à 45 atomes de carbone. Ces cires sont appelées respectivement des alkyldiméthicones et des alcoxydiméthicones. Par ailleurs, ces chaînes alkyle peuvent comporter une ou plusieurs fonctions ester. Comme autres cires utilisables dans l'invention, on peut citer les cires d'origine animale comme la lanoline, la cire d'abeille ; les cires d'origine végétale telles que la cire de carnauba ou de candellila; les cires d'origine minérale, par exemple de paraffine, de lignite ou les cires microcristallines, la cérésine ou l'ozokérite ; les cires synthétiques comme les cires de polyéthylène ; leurs mélanges.

De manière avantageuse, la composition selon l'invention peut notamment contenir de la cire d'abeille.

La composition selon l'invention peut en outre contenir d'autres adjuvants, et en particulier des charges telles que des argiles, des liants tels que la vinylpyrrolidone, des lubrifiants comme les stéarates de polyol ou les stéarates de métaux alcalins ou alcalino-terreux, ainsi que des agents de contrôle du dégagement d'oxygène tels que le carbonate ou l'oxyde de magnésium, des agents colorants ou des agents matifiants comme les oxydes de titane ou encore des agents tensioactifs.

De préférence, la composition selon l'invention comprend au moins un agent tensioactif choisi parmi les tensioactifs anioniques, non ioniques cationiques, amphotères ou zwittérioniques.

A titre d'exemple de tensioactifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkyl(C₆-C₂₄) sulfosuccinates, les alkyl(C₆-C₂₄) éthersulfosuccinates, les alkyl(C₆-C₂₄) amidesulfosuccinates ; les alkyl(C₆-C₂₄) sulfoacétates ; les acyl(C₆-C₂₄) sarcosinates et les acyl(C₆-C₂₄) glutamates. On peut également utiliser les esters d'alkyl(C₆-C₂₄)polyglycosides carboxyliques tels que les alkylglucoside citrates, les alkylpolyglycoside tartrate et les alkylpolyglycoside sulfosuccinates, les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, et palmitique, des acides d'huile de coprah ou d'huile de coprah hydrogénée, et notamment de préférence les sels de sodium, calcium ou magnésium de l'acide stéarique; les acyl-lactylates dont le radical acyle comporte 8 à 20 atomes de carbone. On peut également utiliser les acides d'alkyl D galactoside uroniques et leurs sels, les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, et leurs mélanges.

Les agents tensioactifs non-ioniques utilisables dans les compositions selon l'invention sont des composés bien connus en soi (voir notamment à cet égard "Handbook of Surfactants" par M.R. PORTER, éditions Blackie & Son (Glasgow and London), 1991, pp 116-178). Ainsi, ils peuvent être notamment choisis parmi (liste non limitative) les alcools, les alpha-diols, les alkylphénols polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀-C₁₄) amines ou les oxydes de N-acylaminopropylmorpholine.

Les agents tensioactifs amphotères ou zwitterioniques susceptibles d'être employés dans les compositions selon la présente invention peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

R₂ -CONHCH₂CH₂ -N(R₃)(R₄)(CH₂COO-)

dans laquelle : R₂ désigne un radical alkyle d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₂'-CONHCH₂CH₂-N(B)(C)

dans laquelle :
B représente -CH₂CH₂OX', C représente -(CH₂)z -Y', avec z = 1 ou 2,
X' désigne le groupement -CH₂CH₂-COOH ou un atome d'hydrogène,
Y' désigne -COOH ou le radical -CH₂ - CHOH - SO₃H,
R₂' désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Coco-amphodipropionic acid.

A titre d'exemple on peut citer le cocoamphodiacetate commercialisé sous la dénomination commerciale MIRANOL^{®} C2M concentré par la société RHODIA CHIMIE.

Parmi les tensioactifs cationiques susceptibles d'être employés dans la composition selon l'invention, on peut citer en particulier (liste non limitative): les sels d'amines grasses primaires, secondaires ou tertiaires, éventuellement polyoxyalkylénées; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

La composition selon l'invention peut également contenir un ou plusieurs polymères conditionneurs cationiques ou amphotères anhydres tels que par exemple ceux décrits dans les brevets français N°2788974 et 2788976 et tels que décrits ci-après.

Au sens de la présente invention, l'expression "polymère cationique" désigne tout polymère contenant des groupements cationiques et/ou des groupements ionisables en groupements cationiques.

Les polymères cationiques utilisables conformément à la présente invention peuvent être choisis parmi tous ceux déja connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire.

Ce sont des produits connus. Ils sont notamment décrits dans les brevets français n° 2 505 348 ou 2 542 997. Parmi lesdits polymères, on peut citer :
(1) Les homopolymères ou copolymères dérivés d'esters ou d'amides acryliques ou méthacryliques et comportant au moins un des motifs de formules (II), (III), (IV) ou (V) suivantes: 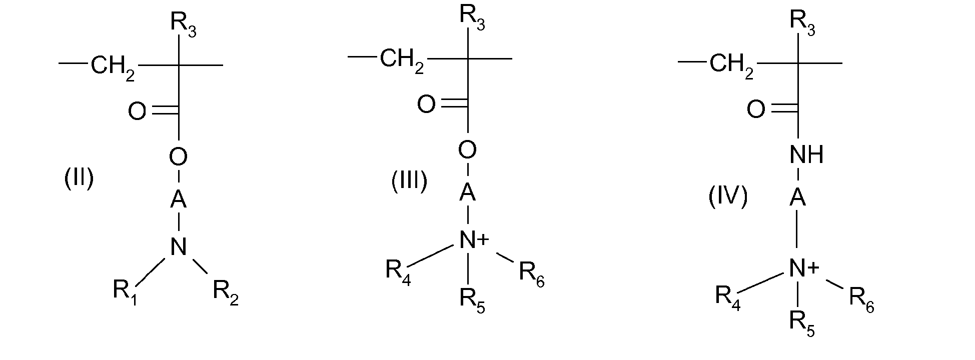 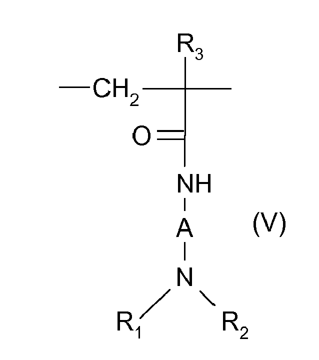 dans lesquelles:
   R₃, identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂, identiques ou différents, représentent hydrogène ou un groupe alkyle ayant de 1 à 6 atomes de carbone et de préférence méthyle ou éthyle;
   X désigne un anion dérivé d'un acide minéral ou organique tel qu'un anion méthosulfate ou un halogénure tel que chlorure ou bromure.
   Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques.
   Ainsi, parmi ces polymères de la famille (1), on peut citer :
   - les copolymères d'acrylamide et de diméthylaminoéthyl méthacrylate quaternisé au sulfate de diméthyle ou avec un hologénure de diméthyle;
   - les copolymères d'acrylamide et de chlorure de méthacryloyloxyéthyl-triméthylammonium décrits par exemple dans la demande de brevet EP-A-080976;
   - le copolymère d'acrylamide et de méthosulfate de méthacryloyloxyéthyl-triméthylammonium;
   - les copolymères vinylpyrrolidone / acrylate ou méthacrylate de dialkylaminoalkyle quaternisés ou non. Ces polymères sont décrits en détail dans les brevets français 2.077.143 et 2.393.573;
   - les terpolymères méthacrylate de diméthyl amino éthyle/vinylcaprolactame/ vinylpyrrolidone;
   - les copolymère vinylpyrrolidone / méthacrylamidopropyl diméthylamine;
   - et les copolymères vinylpyrrolidone / méthacrylamide de diméthylamino-propyle quaternisés.
(2) Les dérivés d'éthers de cellulose comportant des groupements ammonium quaternaire décrits dans le brevet français 1 492 597. Ces polymères sont également définis dans le dictionnaire CTFA comme des ammonium quaternaires d'hydroxyéthylcellulose ayant réagi avec un époxyde substitué par un groupement triméthylammonium.
(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 1 576, tels que les hydroxyalkylcelluloses, comme les hydroxyméthyl-, hydroxyéthyl- ou hydroxypropylcelluloses greffées notamment avec un sel de méthacryloyléthyl triméthylammonium, de méthacrylmidopropyl triméthylammonium, de diméthyl-diallylammonium.
(4) Les polygalactomannanes cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
(5) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
(6) Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508.
(7) Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine.
(8) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
(9) Les cyclopolymères d'alkyl diallyl amine ou de dialkyl diallyl ammonium tels que les homopolymères ou copolymères comportant comme constituant principal de la chaîne des motifs répondant aux formules (VI) ou (VII) : 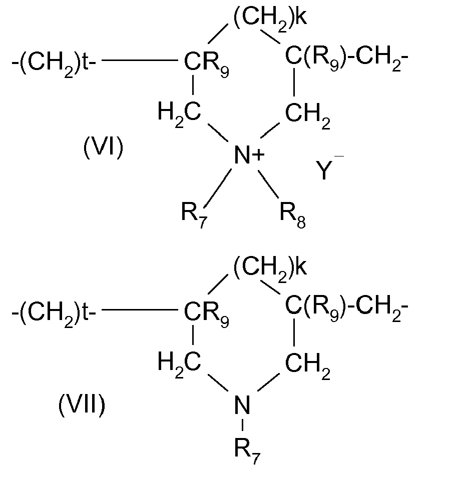 formules dans lesquelles k et t sont égaux à 0 ou 1, la somme k + t étant égale à 1 ; R₉ désigne un atome d'hydrogène ou un radical méthyle ; R₇ et R₈, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C₁-C₄), ou R₇ et R₈ peuvent désigner conjointement avec l'atome d'azote auquel ils sont rattachés, des groupement hétérocycliques, tels que pipéridinyle ou morpholinyle ; R₇ et R₈ indépendamment l'un de l'autre désignent de préférence un groupement alkyle ayant de 1 à 4 atomes de carbone ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
(10) Le polymère de diammonium quaternaire contenant des motifs récurrents répondant à la formule : 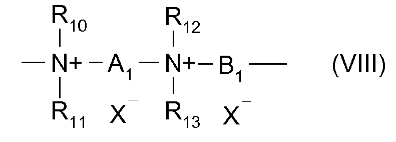 formule (VIII) dans laquelle :
   R₁₀, R₁₁, R₁₂ et R₁₃, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₀, R₁₁, R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁, R₁₂ et R₁₃ représentent un radical alkyle en C₁-C₆ linéaire ou ramifié substitué par un groupement nitrile, ester, acyle, amide ou -CO-O- R₁₄-D ou -CO-NH- R₁₄-D où R₁₄ est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R₁₀ et R₁₂ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement -(CH₂)n-CO-D-OC-(CH₂)n- dans lequel n est compris entre 1 et 100 et de préférence entre 1 et 50, et D désigne :
      a) un reste de glycol de formule : -O-Z-O-, où Z désigne un radical hydrocarboné linéaire ou ramifié ou un groupement répondant à l'une des formules suivantes :

         -(CH₂-CH₂-O)x-CH₂-CH₂-

         -[CH₂-CH(CH₃)-O]y-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent

         -CH₂-CH₂-S-S-CH₂-CH₂- ;
      d) un groupement uréylène de formule : -NH-CO-NH-.
   De préférence, X⁻ est un anion tel que le chlorure ou le bromure.
   Ces polymères ont une masse moléculaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule (IX) suivante: 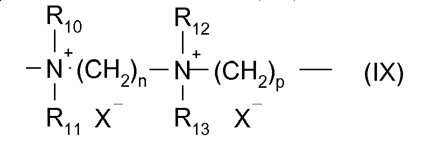 dans laquelle R₁₀, R₁₁, R₁₂ et R₁₃, identiques ou différents, désignent un radical alkyle ou hydroxyalkyle ayant de 1 à 4 atomes de carbone environ, n et p sont des nombres entiers variant de 2 à 20 environ et, X- est un anion dérivé d'un acide minéral ou organique.
(11) Les polymères de poly(ammonium quaternaire) constitués de motifs récurrents de formule (X) : 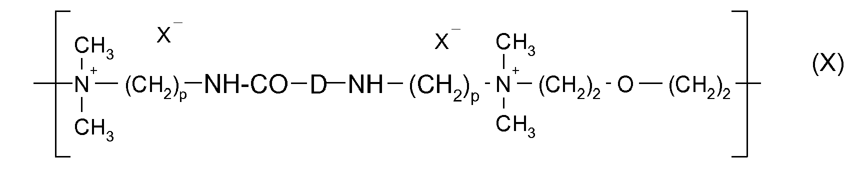 dans laquelle p désigne un nombre entier variant de 1 à 6 environ, D peut être nul ou peut représenter un groupement -(CH₂)ᵣ -CO- dans lequel r désigne un nombre égal à 4 ou à 7, X- est un anion ;
   De tels polymères peuvent être préparés selon les procédés décrits dans les brevets U.S.A. n° 4 157 388, 4 702 906, 4 719 282. Ils sont notamment décrits dans la demande de brevet EP-A-122 324.
(12) Les polymères quaternaires de vinylpyrrolidone et de vinylimidazole.
(13) Les polyamines comme le produit référencé sous le nom de " POLYETHYLENEGLYCOL (15) TALLOW POLYAMINE " dans le dictionnaire CTFA.
(14) Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE^{®} SC 92 " par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE^{®} SC 95 " et " SALCARE^{®} SC 96 " par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Les polymères amphotères utilisables dans les compositions la présente invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;
K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants :
(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537. On peut également citer le copolymère acrylate de sodium / chlorure d'acrylamidopropyl trimethyl ammonium.
   Le composé vinylique peut être également un sel de dialkyldiallylammonium tel que le chlorure de diméthyldiallylammonium.
(2) Les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.
      Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl-acrylamide, le N-tertiooctyl-acrylamide, le N-octylacrylamide, le N-décylacrylamide, le N-dodécylacrylamide ainsi que les méthacrylamides correspondants.
      Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique. Les comonomères basiques préférés sont des méthacrylates d'aminoéthyle, de butyl aminoéthyle, de N,N'-diméthylaminoéthyle, de N-tertio-butyl-aminoéthyle.
(3) Les polyaminoamides réticulés et alcoylés partiellement ou totalement dérivant de polyaminoamides de formule générale :

   ⁅ CO-R₁₉-CO-Z⁆ (XI)

   dans laquelle R₁₉ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical 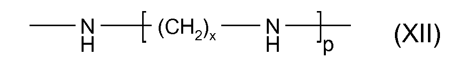 où x=2 et p=2 ou 3, ou bien x=3 et p=2
      ce radical dérivant de la diéthylène triamine, de la triéthylène tétraamine ou de la dipropylène triamine;
   b) dans les proportions de 0 à 40 moles % le radical (XII) ci-dessus, dans lequel x=2 et p=1 et qui dérive de l'éthylènediamine, ou le radical dérivant de la pipérazine : 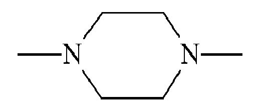
   c) dans les proportions de 0 à 20 moles % le radical -NH-(CH₂)₆-NH-dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels. Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.
      Les alcanes sultones utilisées dans l'alcoylation sont de préférence la propane ou la butane sultone, les sels des agents d'alcoylation sont de préférence les sels de sodium ou de potassium.
(4) Les polymères comportant des motifs zwittérioniques de formule : 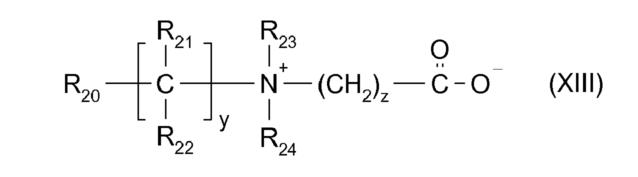 dans laquelle R₂₀ désigne un groupement insaturé polymérisable tel qu'un groupement acrylate, méthacrylate, acrylamide ou méthacrylamide, y et z représentent un nombre entier de 1 à 3, R₂₁ et R₂₂ représentent un atome d'hydrogène, méthyle, éthyle ou propyle, R₂₃ et R₂₄ représentent un atome d'hydrogène ou un radical alkyle de telle façon que la somme des atomes de carbone dans R₂₃ et R₂₄ ne dépasse pas 10.
   Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
   A titre d'exemple, on peut citer le copolymère de méthacrylate de butyle /méthacrylate de diméthylcarboxyméthylammonio-éthyle tel que le produit vendu sous la dénomination DIAFORMER Z301 par la société SANDOZ.
(5) les polymères dérivés du chitosane comportant des motifs monomères répondant aux formules (XIV), (XV), (XVI) suivantes : 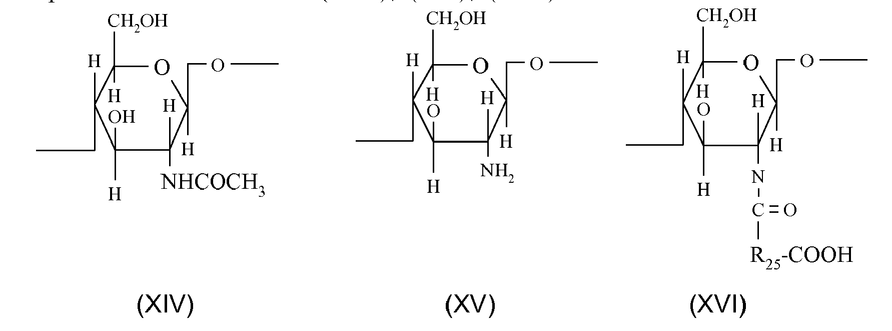 le motif (XIV) étant présent dans des proportions comprises entre 0 et 30%, le motif (XV) dans des proportions comprises entre 5 et 50% et le motif (XVI) dans des proportions comprises entre 30 et 90%, étant entendu que dans ce motif (XVI), R₂₅ représente un radical de formule : 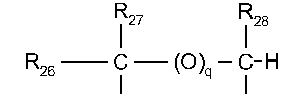 dans laquelle q désigne zéro ou 1 ;
   si q=0, R₂₆, R₂₇ et R₂₈, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux R₂₆, R₂₇ et R₂₈ étant dans ce cas un atome d'hydrogène ;
   ou si q=1, R₂₆, R₂₇ et R₂₈ représentent chacun un atome d'hydrogène, ainsi que les sels formés par ces composés avec des bases ou des acides.
(6) Les polymères dérivés de la N-carboxyalkylation du chitosane comme le N-carboxyméthyl chitosane ou le N-carboxybutyl chitosane.
(7) Les polymères répondant à la formule générale (XVII) tels que ceux décrits par exemple dans le brevet français 1 400 366 : 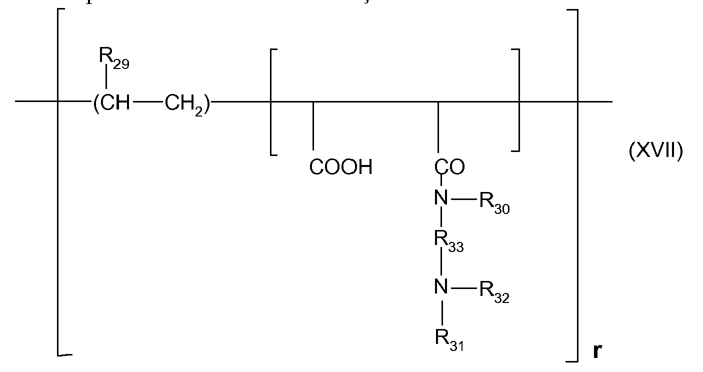 dans laquelle R₂₉ représente un atome d'hydrogène, un radical CH₃O, CH₃CH₂O, phényle, R₃₀ désigne l'hydrogène ou un radical alkyle inférieur tel que méthyle, éthyle, R₃₁ désigne l'hydrogène ou un radical alkyle inférieur tel que méthyle, éthyle, R₃₂ désigne un radical alkyle inférieur tel que méthyle, éthyle ou un radical répondant à la formule : - R₃₃-N(R₃₁)₂, R₃₃ représentant un groupement -CH₂-CH₂- , -CH₂-CH₂-CH₂- , -CH₂-CH(CH₃)- , R₃₁ ayant les significations mentionnées ci-dessus,
   ainsi que les homologues supérieurs de ces radicaux et contenant jusqu'à 6 atomes de carbone,
(8) Des polymères amphotères du type -D-X-D-X- choisis parmi:
   a) les polymères obtenus par action de l'acide chloracétique ou le chloracétate de sodium sur les composés comportant au moins un motif de formule :

      -D-X-D-X-D- (XVIII)

      où D désigne un radical 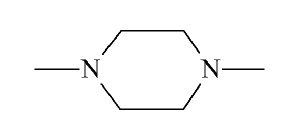 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;
   b) les polymères de formule :

      -D-X-D-X- (XIX)

      où D désigne un radical 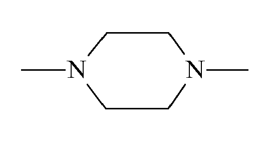 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) Les copolymères alkyl(C₁-C₅)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Parmi tous les polymères cationiques ou amphotères utilisables selon la présente invention, on préfère notamment :
(i)parmi les polymères cationiques :
   - l'homopolymère de chlorure de diméthyldiallylammonium tel que le produit vendu sous la dénomination MERQUAT 100DRY par la société MERCK;
   - les copolymères de chlorure de diméthyldiallylammonium et d'acrylamide tels que les produits vendus sous la dénomination MERQUAT 2200 par la société CALGON;
   - les polymères de type poly(ammonium quaternaire) préparés et décrits dans le brevet français 2 270 846, constitués de motifs récurrents de formules (W) et (U) suivantes : 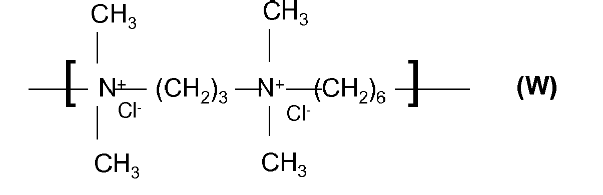 et notamment ceux dont le poids moléculaire, déterminé par chromatographie par perméation de gel, est compris entre 9500 et 9900; 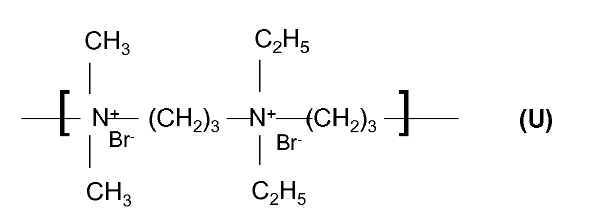 et notamment ceux dont le poids moléculaire, déterminé par chromatographie par perméation de gel, est d'environ 1200;
   - les polymères de type poly(ammonium quaternaire) de la famille (11) et de formule (X) dans laquelle X- désigne le Chlore, et notamment ceux dont la masse moléculaire moyenne en poids est inférieure à 100 000, de préférence inférieure ou égale à 50 000;
(ii) parmi les polymères amphotères:
   - le copolymère chlorure de diméthyldiallylammonium/acide acrylique (80/20) commercialisé sous la dénomination MERQUAT 280 DRY par la société CALGON (dénomination CTFA : POLYQUATERNIUM 22);
   - le copolymère chlorure de diméthyldiallylammonium/acide acrylique (95/5) commercialisé sous la dénomination MERQUAT 295 DRY par la société CALGON (dénomination CTFA : POLYQUATERNIUM 22);
   - le copolymère de chlorure de méthacrylamidopropyltrimonium, d'acide acrylique et d'acrylate d'éthyle, commercialisé sous la dénomination MERQUAT 2001 par la société CALGON (dénomination CTFA : POLYQUATERNIUM 47); et
   - le terpolymère acrylamide/chlorure de diméthyldiallylammonium/acide acrylique, commercialisé sous la dénomination MERQUAT PLUS 3330 DRY par la société CALGON (dénomination CTFA : POLYQUATERNIUM 39).

Lorsqu'ils ils sont présents dans les compositions selon la présente invention, les polymères cationiques et/ou amphotères sont présents dans une proportion pondérale totale inférieure ou égale à 20% par rapport au poids total de la composition, et de préférence inférieure ou égale à 8%.

De préférence la composition anhydre sous forme de pâte est constituée par mélange d'au moins une poudre et d'au moins un liquide inerte.

La composition anhydre selon l'invention peut être préparée en dispersant, sous action mécanique, l'ensemble des composés pulvérulents dans le liquide inerte organique, dans lequel on a préalablement dispersé ou mélangé les autres composés liquides de la composition.

On peut également préparer la composition par extrusion, en introduisant les phases liquides et solides de la composition dans un extrudeur, puis en les mélangeant à une température inférieure à 25°C à l'aide d'un système bi-vis co-rotatives composé d'éléments de transport et de malaxage.

La composition anhydre selon l'invention est utilisée pour la préparation d'une composition prête à l'emploi qui résulte du mélange extemporané de ladite composition anhydre avec une composition aqueuse contenant ou non du peroxyde d'hydrogène.

Ce mélange se fait de préférence immédiatement avant l'application du produit sur les cheveux.

Selon un mode de réalisation avantageux, la composition prête à l'emploi résulte du mélange extemporané de ladite composition anhydre avec une composition aqueuse ne contenant pas de peroxyde d'hydrogène.

La composition anhydre sous forme de pâte selon l'invention est ainsi mélangée avec environ 0,5 à 10 équivalents en poids d'une composition aqueuse qui peut être une solution, une émulsion ou un gel.

Lorsque ladite composition aqueuse comprend du peroxyde d'hydrogène, elle présente une concentration pondérale allant de 2 à 12% en peroxyde d'hydrogène, et de préférence de 2 à 6%. Elle peut contenir en outre des agents stabilisants du peroxyde d'hydrogène tels que notamment le pyrophosphate de sodium, le stannate de sodium et le salicylate de sodium.

Lorsque ladite composition aqueuse comprend du peroxyde d'hydrogène, elle présente de préférence un pH inférieur à 7. Le pH acide garantit la stabilité du peroxyde d'hydrogène dans la composition. I1 peut être obtenu à l'aide d'agents acidifiants tels que par exemple l'acide chlorhydrique, l'acide acétique, l'acide éthydronique, l'acide phosphorique, l'acide lactique ou l'acide borique, et il peut être ajusté classiquement par ajout soit d'agents basifiants, tels que par exemple, l'ammoniaque, la monoéthanolamine, la diéthanolamine, la triéthanolamine, l'isopropanolamine, le 1,3-diamino-propane, un (bi)carbonate alcalin ou d'ammonium, un carbonate organique tel que le carbonate de guanidine, ou bien encore un hydroxyde alcalin, tous ces composés pouvant bien entendu être pris seuls ou en mélange.

Quelle contienne ou non du peroxyde d'hydrogène, la composition aqueuse peut contenir en outre des agents conservateurs, des colorants, des parfums, des agents antimousse, ainsi que des agents séquestrants tels que par exemple l'acide éthylènediamine tétraacétique (EDTA) ou le Pentasodium Pentetate (dénomination CTFA).

Bien entendu, l'homme de l'art veillera à choisir le ou les éventuels composés complémentaires mentionnés ci-avant, de manière telle que les propriétés avantageuses attachées intrinsèquement à la composition anhydre sous forme de pâte ou à la composition de décoloration prête à l'emploi selon l'invention ne soient pas, ou substantiellement pas, altérées par la ou les adjonctions envisagées.

Le pH de la composition de décoloration prête à l'emploi est de préférence compris entre 7 et 12, bornes incluses. I1 est de préférence compris entre 7,5 et 11, bornes incluses.

De préférence, le procédé de décoloration selon l'invention consiste à mélanger immédiatement avant emploi une composition anhydre telle que décrite ci-avant avec une composition aqueuse telle que décrite ci-avant, contenant ou ne contenant pas du peroxyde d'hydrogène, à appliquer la composition de décoloration prête à l'emploi ainsi obtenue sur la zone des fibres kératiniques humaines (sèches ou humides) à décolorer, et à la laisser agir pendant un temps de pose suffisant pour obtenir la décoloration recherchée, variant de préférence, de 1 à 60 minutes environ, et plus préférentiellement de 10 à 45 minutes environ, à éliminer le mélange décolorant par rinçage à l'eau, éventuellement suivi d'un lavage avec un shampooing, puis éventuellement d'un séchage.

Selon un mode de réalisation particulièrement avantageux, ladite composition aqueuse est de l'eau.

La présente invention a aussi pour objet un dispositif à plusieurs compartiments, ou "kit", pour la décoloration des fibres kératiniques humaines et plus particulièrement des cheveux, caractérisé par le fait qu'il comporte au moins deux compartiments dont l'un contient une composition anhydre telle que décrite ci-avant, et l'autre une composition aqueuse telle que décrite ci-avant, contenant ou ne contenant pas du peroxyde d'hydrogène.

La présente invention a enfin pour objet l'utilisation d'une composition anhydre telle que décrite ci-avant pour la préparation d'une composition de décoloration des fibres kératiniques prête à l'emploi, par mélange avec une composition aqueuse contenant ou ne contenant pas de peroxyde d'hydrogène

De préférence, dans cette utilisation on n'emploie pas de composition aqueuse de peroxyde d'hydrogène. De manière préférée, ladite préparation est réalisée par mélange de la composition anhydre selon l'invention, avec une composition aqueuse ne contenant pas de peroxyde d'hydrogène.

Les exemples suivants sont donnés à titre illustratif de la présente invention, et ne sauraient en limiter la portée.

### EXEMPLES

Dans les exemples suivants, toutes les quantités sont indiquées en pour cent en poids de matière active par rapport au poids total de la composition, sauf indication contraire.

Deux compositions, sont préparées à partir des ingrédients indiqués dans le tableau ci-dessous.

### Exemple 1 :

La composition B selon l'invention comprend 10% en poids d'un complexe de polyvinylpyrrolidone (PVP), et de peroxyde d'hydrogène (H₂O₂), tandis que la composition A comparative est une composition classique, ne comprenant pas un tel complexe.

| Ingrédients (% en poids MA) | Composition A comparative | Composition B selon l'invention |
|---|---|---|
| Persulfate de potassium | 34,75 | 34,75 |
| Persulfate de sodium | 6 | 6 |
| Métasilicate de sodium | 3,4 | 3,4 |
| Disilicate de sodium | 14,2 | 14,2 |
| Chlorure d'ammonium | 4,2 | 4,2 |
| EDTA | 0,2 | 0,2 |
| Dioxyde de titane | 1 | 0 |
| Lauryl sulfate de sodium (EMPICOL LX/N de HUNTSMAN) | 4 | 1 |
| Stéarate de magnésium | 2 | 0 |
| Polyuréthane (RHEOLATE FX11000 de ELEMENTIS) | 2 | 0 |
| Carboxyméthyl amidon (PRIMOJEL de DMV INTERNATIONAL) | 2 | 0 |
| Silice colloïdale (LEVILITE STANDARD de CECA) | 1 | 0 |
| Complexe PVP-H₂O₂ (PEROXYDONE K-30 d'ISP) | 0 | 10 |
| Huile minérale (MARCOL 82 d'EXXON-MOBIL) | 1 | 1 |
| Gomme de xanthane (KELTROL CGBT de CP KELCO) | 1,4 | 2,4 |
| Cire d'abeille | 1,2 | 1,2 |
| Myristate d'isopropyle | 21,6 | 21,6 |
| Céramide (MEXANYL GZ de CHIMEX) | 0,01 | 0,01 |
| Bleu d'outremer 09 | 0,04 | 0,04 |

| | | |
|---|---|---|
| MA = matière active | | |

Les compositions A et B sont mélangées dans un rapport 1 + 1,5 en poids avec une composition aqueuse de peroxyde d'hydrogène à 12% en poids, puis les compositions prêtes à l'emploi ainsi formées sont appliquées sur des mèches de cheveux châtain avec un rapport de bain de 10, pendant un temps de pose de 40 minutes, à une température de 27°C. Les mèches après traitement ont été ensuite rincées à l'eau, shampooinées puis séchées.

La demanderesse a effectué des mesures colorimétriques sur les mèches de cheveux décolorées ainsi obtenues.

Le pouvoir décolorant des compositions A et B a été mesuré avec un colorimètre MINOLTA CM 2002 dans le système international CIE L*a*b*.

Les valeurs obtenues sont les suivantes :

| | L* | a* | b* |
|---|---|---|---|
| Composition A | 49,2 | 12,1 | 29 |
| Composition B | 53,3 | 11,5 | 30,4 |

Dans ces valeurs, L^{*} représente la clarté de la nuance obtenue. La valeur L^{*} est comprise entre 0 et 100. Plus cette valeur est élevée, plus la nuance est claire.

La mèche obtenue à partir de la composition B est significativement plus claire que celle obtenue à partir de la composition A.

Ces résultats montrent que les performances éclaircissantes ou décolorantes obtenues avec la composition l'invention sont supérieures à celles de l'art antérieur.

### Exemple 2 :

| Ingrédients (% en poids MA) | Composition C selon l'invention |
|---|---|
| Persulfate de potassium | 33 |
| Persulfate de sodium | 6 |
| Metasilicate de sodium | 3,4 |
| Disilicate de sodium | 12 |
| Chlorure d'ammonium | 4,2 |
| EDTA | 0,2 |
| Lauryl sulfate de sodium (EMPICOL LX/N de HUNTSMAN) | 1 |
| Complexe PVP-H₂O₂ (PEROXYDONE K-30 d'ISP) | 20 |
| Huile minérale (MARCOL 82 d'EXXON-MOBIL) | 17 |
| Gomme de xanthane (KELTROL CGBT de CP KELCO) | 2 |
| Cire d'abeille | 1,2 |

| | |
|---|---|
| MA = matière active | |

La composition C est mélangée dans un rapport 1 + 1,5 en poids avec de l'eau puis la composition prête à l'emploi ainsi formée est appliquée sur des mèches de cheveux châtain avec un rapport de bain de 10, pendant un temps de pose de 40 minutes, à une température de 27°C. On obtient un bon effet d'éclaircissement.

## Revendications

1. Composition anhydre sous forme de pâte pour la décoloration des fibres kératiniques, comprenant un ou plusieurs sels peroxygénés, un ou plusieurs agents alcalins, et un ou plusieurs complexes de peroxyde d'hydrogène et d'un polymère comprenant à titre de monomère au moins un monomère hétérocyclique vinylique.

2. Composition selon l'une quelconque des revendications précédentes, **caractérisée en ce que** le monomère hétérocycle vinylique est choisi parmi les monomères comprenant un hétérocycle ayant 4 à 6 chaînons, éventuellement substitué, éventuellement condensé à un cycle benzénique, comportant de 1 à 4 hétéroatomes intracycliques indentiques ou non ; le nombre d'hétéroatomes intracycliques étant inférieur à celui des chaînons de l'hétérocycle ; le ou les hétéroatomes étant choisis parmi le soufre, l'oxygène, l'azote.

3. Composition selon l'une quelconque des revendications précédentes, **caractérisée en ce que** le monomère hétérocyclique vinylique est choisi parmi les monomères N-vinyliques.

4. Composition selon l'une quelconque des revendications précédentes, **caractérisée en ce que** le monomère hétérocyclique vinylique est choisi parmi la N-vinylpyrrolidone, le vinylcaprolactame, la N-vinylpipéridone, la N vinyl 3-morpholine, la N-vinyl-4-oxazolinone, la 2-vinylpyridine, la 4-vinylpyridine, la 2-vinylquinoline, le1-vinylimidazole, le 1-vinylcarbazole, éventuellement substitués.

5. Composition selon l'une quelconque des revendications précédentes, **caractérisée en ce que** le monomère hétérocyclique vinylique est la vinylpyrrolidone.

6. Composition selon l'une quelconque des revendications précédentes, **caractérisée en ce que** le polymère comprend au moins un autre monomère choisi parmi l'acétate de vinyle, les acides (méth)acryliques, les amides (méth)acryliques, les esters d'alkyle en C₁-C₄ d'acide (méth)acryliques, les esters d'alkyle en C₁-C₄ d'acide (méth)acrylique, substitués ou non.

7. Composition selon l'une quelconque des revendications précédentes, **caractérisée en ce que** le polymère contenant à titre de monomère, au moins un monomère hétérocyclique vinylique est un homopolymère.

8. Composition selon l'une quelconque des revendications précédentes, **caractérisée en ce que** le complexe de peroxyde d'hydrogène et de polymère comprend de 10 à 30 % en poids de peroxyde d'hydrogène, par rapport au poids total du complexe.

9. Composition selon l'une quelconque des revendications précédentes, **caractérisée en ce que**, dans le complexe de peroxyde d'hydrogène et de polymère, le rapport molaire entre le ou les monomère(s) hétérocyclique(s) vinylique(s) et le peroxyde d'hydrogène va de 0,5 à 2.

10. Composition selon l'une quelconque des revendications précédentes, **caractérisée en ce qu'**elle contient de 0,1 à 50 % en poids de complexe de peroxyde d'hydrogène et de polymère, par rapport au poids total de la composition.

11. Composition selon l'une quelconque des revendications précédentes, **caractérisée en ce qu'**elle contient un ou plusieurs sels peroxygénés choisis parmi les persulfates, les perborates et les percarbonates d'ammonium ou de métaux alcalins ainsi que le peroxyde de magnésium et les mélanges de ces composés.

12. Composition selon l'une quelconque des revendications précédentes, **caractérisée en ce que** le ou les sel(s) peroxygéné(s) sont présents à une concentration allant de 10 à 70% en poids, par rapport au poids total de la composition.

13. Composition selon l'une quelconque des revendications précédentes, **caractérisée en ce qu'**elle contient un ou plusieurs agents alcalins, choisis parmi l'ammoniaque, les silicates, les métasilicates, les phosphates, les hydrogénophosphates, les carbonates et les hydrogénocarbonates de métaux alcalins ou alcalino-terreux.

14. Composition selon l'une quelconque des revendications précédentes, **caractérisée en ce que** le ou les agent(s) alcalin(s) sont présents à une concentration allant de 0,01 à 40% en poids, par rapport au poids total de la composition.

15. Composition selon l'une quelconque des revendications précédentes, **caractérisée en ce qu'**elle contient également un ou plusieurs sels d'ammonium.

16. Composition selon la revendication précédente, **caractérisée en ce que** le ou les sels d'ammonium sont présents à une concentration allant de 0,01 à 40% en poids, de préférence de 0,1 à 30% en poids, par rapport au poids total de la composition.

17. Composition selon l'une quelconque des revendications précédentes, **caractérisée en ce qu'**elle contient un ou plusieurs liquide(s) inerte(s) organique(s).

18. Composition selon la revendication précédente, **caractérisée en ce que** le liquide inerte organique est choisi dans le groupe formé par les polydécènes de formule C₁₀ₙH_{[(20n)+2]} avec n variant de 3 à 9, les esters d'alcools gras ou d'acides gras, les esters ou di-esters de sucres et d'acides gras en C₁₂-C₂₄, les éthers cycliques ou les esters cycliques, les huiles de silicone, les huiles minérales ou les huiles végétales, et leurs mélanges.

19. Composition selon la revendication précédente, **caractérisée en ce que** le liquide inerte organique est choisi parmi les polydécènes, les esters d'alcools gras ou d'acides gras, les huiles minérales et leurs mélanges.

20. Composition selon l'une quelconque des revendications 17 à 19, **caractérisée en ce que** le ou les liquide(s) inerte(s) organique(s) sont présents à une concentration allant de 15 à 35% en poids par rapport au poids total de la composition.

21. Composition selon la revendication précédente, **caractérisée en ce que** le ou les liquide(s) inerte(s) organique(s) sont présents à une concentration allant de 18 à 30% en poids par rapport au poids total de la composition.

22. Composition selon l'une quelconque des revendications précédentes, **caractérisée en ce qu'**elle comprend également au moins un polymère amphiphile non ionique comportant au moins une chaîne grasse.

23. Composition selon l'une quelconque des revendications précédentes, **caractérisée en ce qu'**elle comprend également au moins un polymère amphiphile anionique comportant au moins une chaîne grasse.

24. Composition selon l'une quelconque des revendications précédentes, **caractérisée en ce qu'**elle comprend également au moins un polymère épaississant hydrosoluble sans chaînes grasses.

25. Composition selon l'une quelconque des revendications précédentes, **caractérisée en ce qu'**elle comprend également au moins un agent tensioactif choisi parmi les tensioactifs anioniques, non ioniques cationiques, amphotères ou zwittérioniques.

26. Composition selon l'une quelconque des revendications précédentes, **caractérisée en ce qu'**elle comprend également un ou plusieurs polymères conditionneurs cationiques ou amphotères anhydres.

27. Composition prête à l'emploi, résultant du mélange extemporané d'une composition anhydre selon l'une quelconque des revendications 1 à 26 avec une composition aqueuse contenant ou non du peroxyde d'hydrogène.

28. Composition selon la revendication précédente, **caractérisée en ce que** la composition aqueuse ne contient pas de peroxyde d'hydrogène.

29. Procédé de décoloration des fibres kératiniques humaines, consistant à mélanger immédiatement avant emploi une composition anhydre selon l'une quelconque des revendications 1 à 26 avec une composition aqueuse contenant ou ne contenant pas du peroxyde d'hydrogène, à appliquer la composition de décoloration prête à l'emploi ainsi obtenue sur la zone des fibres kératiniques humaines à décolorer, et à la laisser agir pendant un temps de pose suffisant pour obtenir la décoloration recherchée, à éliminer le mélange décolorant par rinçage à l'eau, éventuellement suivi d'un lavage avec un shampooing, puis éventuellement d'un séchage.

30. Procédé selon la revendication précédente, **caractérisé en ce que** ladite composition aqueuse est de l'eau.

31. Dispositif à plusieurs compartiments, ou "kit", pour la décoloration des fibres kératiniques humaines, **caractérisé par le fait qu'**il comporte au moins deux compartiments dont l'un contient une composition anhydre selon l'une quelconque des revendications 1 à 26, et l'autre une composition aqueuse contenant ou ne contenant pas du peroxyde d'hydrogène.

32. Utilisation d'une composition anhydre selon l'une quelconque des revendications 1 à 26 pour la préparation d'une composition de décoloration des fibres kératiniques prête à l'emploi, par mélange avec une composition aqueuse ne contenant pas de peroxyde d'hydrogène.

33. Utilisation d'une composition anhydre selon l'une quelconque des revendications 1 à 26 pour la préparation d'une composition de décoloration des fibres kératiniques prête à l'emploi, par mélange avec une composition aqueuse contenant du peroxyde d'hydrogène.
